(19) Europäisches Patentamt
European Patent Office
Office européen des brevets

(11) Veröffentlichungsnummer: **0 245 831**
**A2**

(12) # EUROPÄISCHE PATENTANMELDUNG

(21) Anmeldenummer: 87106832.6

(22) Anmeldetag: 12.05.87

(51) Int.Cl.³: C 07 D 498/04
C 07 F 7/18, A 61 K 31/435
//(C07D498/04, 263:00, 221:00)

(30) Priorität: 14.05.86 DE 3616180

(43) Veröffentlichungstag der Anmeldung:
19.11.87 Patentblatt 87/47

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Hoppe, Hans-Ullrich, Dr.
Habichtsweg 4
D-6238 Hofheim am Taunus(DE)

(72) Erfinder: Grabley, Susanne, Dr.
Hölderlinstrasse 7
D-6240 Königstein/Taunus(DE)

(72) Erfinder: Voelskow, Hartmut, Dr.
Akazienstrasse 22
D-6234 Hattersheim am Main(DE)

(72) Erfinder: Schlingmann, Merten, Dr.
Schneidhainer Strasse 32a
D-6240 Königstein/Taunus(DE)

(72) Erfinder: Kraemer, Hans Peter, Dr.
Birkenweg 16
D-3550 Marburg(DE)

(72) Erfinder: Wiesner, Matthias, Dr.
Im Münchfeld 8
D-6500 Mainz(DE)

(72) Erfinder: Thiem, Joachim, Prof.Dr.
Asbeckweg 31
D-4400 Münster(DE)

(54) **Diels-Alder-Addukte aus Streptazolin und Naphthochinonen und ihre Oxidationsprodukte, Verfahren zu ihrer Herstellung und ihre Verwendung.**

(57) Streptazolin wird in einer Diels-Alder-Reaktion mit Naphthochinonen umgesetzt und das Addukt anschließend oxidiert. Die entstandenen Verbindungen zeigen cytotoxische Aktivität, insbesondere gegen Leukämiezellen, und wirken antimikrobiell sowie gegen Protozoen.

EP 0 245 831 A2

Croydon Printing Company Ltd

Diels-Alder-Addukte aus Streptazolin und Naphthochinonen und ihre Oxidationsprodukte, Verfahren zu ihrer Herstellung und ihre Verwendung

Krebs-Erkrankungen stellen derzeit eine der größten Herausforderungen an die Medizin dar. Chirurgischoperative Eingriffe bzw. Strahlentherapie reichen bei der Mehrzahl der Krebspatienten nicht mehr aus, da durch Metastasenbildung eine lokale Bekämpfung des Tumors sinnlos ist. Nur die Krebs-Chemotherapie bietet in diesem Stadium der Krankheit noch eine Chance auf Heilung. Daher besteht ein gesteigertes Interesse an neuen geeigneten Verbindungen mit möglichst geringen Nebenwirkungen. Nach einer Zusammenstellung des National Cancer Institute, USA, befinden sich zur Zeit etwa 50 Naturstoffe bzw. Naturstoffderivate zur Krebschemotherapie im klinischen Einsatz und etwa weitere 100 in den verschiedenen Phasen der klinischen Entwicklung. Ein besonderes Interesse besitzen dabei die Anthracycline, die hinsichtlich ihrer cytotoxischen Wirksamkeit seit ca. 25 Jahren systematisch untersucht werden.

In der vorliegenden Erfindung werden Verbindungen mit cytotoxischen Eigenschaften beschrieben, die durch Derivatisierung von Streptazolin hergestellt werden.

Streptazolin (Verbindung der Formel IV, $R^3$ = H) wurde von Zähner et al. [Helv. Chim. Acta <u>64</u>, 1752 (1981)] als sekundärer Metabolit aus Streptomyces viridochromogenes isoliert und von Keller-Schierlein et al. [Helv. Chim Acta <u>65</u>, 1432, (1982)] in seiner Struktur aufgeklärt. Die Totalsynthese von Streptazolin wurde von Kozikowski et al. beschrieben [JACS <u>107</u>, 1763, (1984)].

Streptazolin ist nur ein sehr schwaches Antibiotikum und hat keine cytotoxische Wirkung. Durch Umsetzung der Verbindung oder ihrer Derivate entsprechend der Formel mit Naphthochinonen enstehen Produkte, die sich weiter in 1,4-Dihydroanthrachinon- bzw. Anthrachinonderivaten oxidieren lassen. Sowohl die Diels-Alder-Addukte als auch ihre Oxidationsprodukte besitzen cytotoxische und antimikrobielle Eigenschaften bzw. Wirkung gegen Protozoen.

Die Erfindung betrifft somit:

1. Die Verbindung der allgemeinen Formel I,

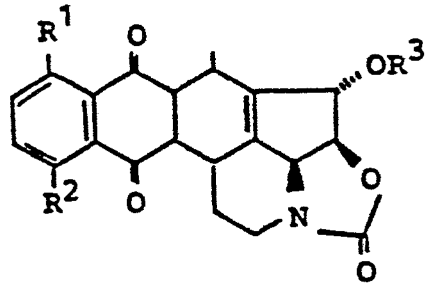

I

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten.

2. Die Verbindung der allgemeinen Formel II,

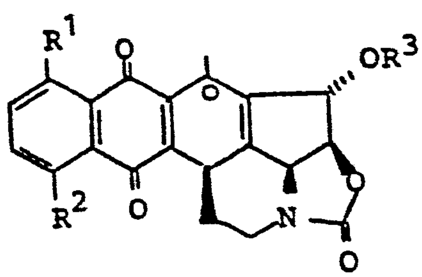

II

bei der an der mit "o" gekennzeichneten Position die beiden möglichen Epimeren auftreten und in der $R^1$ und $R^2$ unabhängig voneinander Wasserstoff, Hydroxyl, Methoxy, O-Acetyl oder O-Benzyl und $R^3$ Wasserstoff, Acetyl,

Trimethylsilyl, Dimethyltertiärbutylsilyl oder Glukosyl bedeuten.

3. Die Verbindung der allgemeinen Formel III,

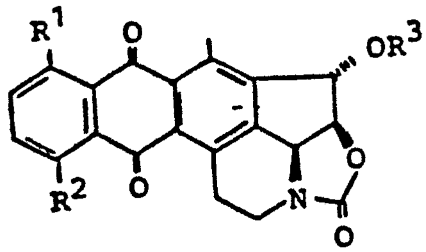

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten.

4. Ein Verfahren zur Herstellung der Verbindung der allgemeinen Formel I, das dadurch gekennzeichnet ist, daß man die Verbindung der Formel IV

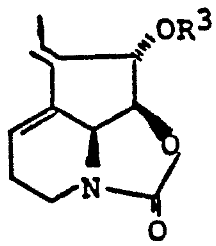

IV

in der $R^3$ Wasserstoff, Acetyl, Trimethylsilyl, Dimethyltertiärbutylsilyl oder Glukosyl bedeutet
mit der Verbindung der allgemeinen Formel V,

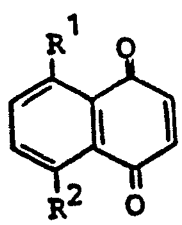

V

in der $R^1$ und $R^2$ unabhängig voneinander Wasserstoff, Hydroxyl, Methoxy, O-Acetyl oder O-Benzyl bedeuten, miteinander zur Reaktion bringt.

5. Ein Verfahren zur Herstellung der Verbindungen der allgemeinen Formeln II und III, das dadurch gekennzeichnet ist, daß die Verbindung der allgemeinen Formel I oxidiert wird.

6. Die Verwendung der Verbindung der allgemeinen Formeln I und/oder II und/oder III als cytotoxisch wirkendes Agens.

7. Die Verwendung der Verbindung der allgemeinen Formeln I und/oder II und/oder III als antimikrobiell und gegen Protozoen wirkendes Agens.

8. Die Verbindung der allgemeinen Formel I und/oder II und/oder III zur Verwendung als Heilmittel.

Streptazolin $R^3$ = H kann nach an sich bekannten Methoden aus Streptomyceten gewonnen werden. Diese Verbindung und dessen Derivate, die in der allgemeinen Formel IV dargestellt sind, werden als konjugiertes Dien in einer Diels-Alder-Reaktion an das Dienophil der allgemeinen Formel V addiert. Die Reaktion läuft im verschlossenen Gefäß bei erhöhten Temperaturen von ungefähr 60 bis 140°C in inerten Lösungsmitteln ab und ist nach einem Verlauf von 4 bis 15 Stunden beendet. Geeignete Lösungsmittel sind beispielsweise Toluol, Nitrotoluol, Xylol, Benzol, Tetrahydrofuran oder Chloroform. Besonders gute Ergebnisse erzielt man, wenn die Reaktionspartner bei 100 bis 120°C in Toluol zusammengebracht werden. Alternativ können die Reaktionspartner IV und V auch in Methylenchlorid oder Chloroform unter Zusatz von Lewis-Säuren wie beispielsweise Aluminiumtrichlorid oder Titantetrachlorid bei Temperaturen zwischen -20 bis +10°C umgesetzt werden, wenn $R^1$ und $R^2$ nicht Hydroxyl bzw. $R^3$ nicht Wasserstoff sind. Die Reaktionsgemische werden anschließend

von den betreffenden Lösungsmitteln und Katalysatoren befreit. Die Verbindung I kann mit Hilfe chromatographischer Methoden gereinigt werden.

Die Verbindung der allgemeinen Formel I dient als Zwischenprodukt zur Herstellung der Verbindungen der allgemeinen Formeln II und III. Diese erhält man durch Oxidation der regio- bzw. diastereomeren Verbindung der allgemeinen Formel I in alkalischen Mischungen aus ($C_1$-$C_4$)-Alkoholen und Wasser. Besonders bevorzugt verläuft die Oxidation im allgemeinen mit Luftsauerstoff bei Temperaturen von 10 bis 40°C in einer Lösung aus Wasser und Methanol, deren Mischungsverhältnis im Bereich 20:1 bis 1:20 liegt. Mit einer Lauge, insbesondere Natronlauge, Kalilauge und Carbonatlösungen, Pyridin bzw. Ammoniak wird der pH auf einen Wert zwischen 9 und 14 eingestellt. Bei hydrolyseempfindlichen Verbindungen I (z.B. $R^1$ = $R^2$ = O-Acetyl und $R^3$ = Acetyl) kann die Oxidation auch in wasserfreiem Methanol mit Pyridin und reinem Sauerstoff erfolgen.

Unter den beschriebenen Bedingungen bildet sich bevorzugt, wie durch NOE-Messungen festgestellt werden konnte, die Verbindung II in der dargestellten Konfiguration, wobei an der mit "o" gekennzeichneten Position beide Epimere auftreten können.

Es konnte nachgewiesen werden, daß die Verbindung der allgemeinen Formel II kein Zwischenprodukt auf dem Weg zur Verbindung der allgemeinen Formel III darstellt, da sich III nicht durch nochmalige Anwendung der oben beschriebenen oxidierenden Bedingungen aus II herstellen läßt.

Die Verbindungen der allgemeinen Formeln I, II und III zeigen cytotoxische Wirkung, insbesondere gegen Leukämiezellen. Die Verbindungen zeigen ferner antimikrobielle, insbesondere antifungische Wirkung, besonders bevorzugt gegen Candida albicans sowie Wirkung gegen Protozoen, insbesondere gegen Trichomonas vaginalis.

0245831

In den folgenden Beispielen wird die Erfindung detailliert erläutert. Die Prozentangaben beziehen sich auf das Gewicht.

Beispiel 1

400 mg Streptazolin ($R^3$ = H) (1,93 mMol) und 367 mg Naphthazarin ($R^1$=$R^2$=OH) (1.93 mMol) werden in 100 ml Toluol 10 bis 12 Stunden bei 100 bis 120°C in einem verschlossenen Gefäß gerührt. Anschließend wird die Reaktionsmischung im Vakuum vom Lösungsmittel befreit und der Rückstand an Kieselgel 60 (Fa. Merck) in Chloroform/Methanol (95/5) chromatographiert. Neben nicht umgesetztem Streptazolin und Naphthazarin erhält man 500 mg eines Gemisches der farblosen und bei 366 nm stark fluoreszierenden Diels-Alder-Addukte. Die Umsetzung kann vervollständigt werden, wenn das Dienophil in doppeltem Überschuß eingesetzt wird.

Beispiel 2

Man verfährt gemäß Beispiel 1, setzt jedoch Naphthochinon ($R^1$ = $R^2$ = H) statt Naphthazarin ein. Man erhält die entsprechenden Diels-Alder-Addukte in analogen Ausbeuten.

Beispiel 3

Die aus Beispiel 1 erhaltenen Diels-Alder-Addukte werden in einer Mischung aus 20 ml Wasser, 60 ml Methanol und 20 ml 2N Natronlauge über einen Zeitraum von 10 bis 15 Minuten an der Luft bei Raumtemperatur gerührt. Anschließend wird diese Lösung mit wäßriger Salzsäure auf den pH-Wert 1 eingestellt und 3 mal mit Chloroform extrahiert. Die organische Phase wird über Natriumsulfat getrocknet und das Lösungsmittel im Vakuum entfernt.

Man erhält 450 mg (59 % bezogen auf Streptazolin) der Verbindung der Formel II, in der $R^1$ und $R^2$ Hydroxyl und $R^3$ Wasserstoff bedeuten, und 30 mg ( 4 %, bezogen auf

Streptazolin) der Verbindung der Formel III, in der $R^1$, $R^2$ und $R^3$ die genannte Bedeutung haben. Abbildung 1 zeigt das $^1H$-NMR-Spektrum der Verbindung II mit $R^1$ = $R^2$ = Hydroxyl und $R^3$ = Wasserstoff.

Beispiel 4

Man verfährt gemäß Beispiel 3, setzt jedoch die aus Beispiel 2 erhaltenen Diels-Alder-Addukte ein. Man erhält 233 mg (31 % bezogen auf Streptazolin) der Verbindung der Formel II, in der $R^1$, $R^2$ und $R^3$ Wasserstoff bedeuten, und 149 mg (20 % bezogen auf Streptazolin) der Verbindung III, in der $R^1$, $R^2$ und $R^3$ die genannte Bedeutung haben. Abbildungen 2 und 3 zeigen die $^1H$-NMR-Spektren der genannten Verbindungen.

Beispiel 5

400 mg Streptazolin-dimethyl-tertiärbutyl-silylether (1,24 mmol) und 196,11 mg (1,24 mmol) 1,4-Naphthochinon und 165,34 mg $AlCl_3$ werden in 40 ml Methylenchlorid bei 0°C 30 Minuten in einem geschlossenen Gefäß gerührt. Anschließend wird die Reaktionsmischung zwischen $H_2O$ und Methylenchlorid verteilt und die organische Phase über Natriumsulfat getrocknet. Nach dem Entfernen des Lösungsmittels im Vakuum, wird der Rückstand an Kieselgel 60 in Chloroform/Methanol (95:5) chromatographiert. Man erhält 535 mg Diels-Alder-Addukte. (90 % bezogen auf Streptazolin-dimethyl-tertiärbutyl-silylether.)

Beispiel 6

Die gemäß Beispiel 5 erhaltenen Diels-Alder-Addukte werden in einer Mischung aus 200 ml Methanol und 15 ml 2N Natronlauge über einen Zeitraum von 15 Minuten an der Luft bei Raumtemperatur gerührt. Anschließend wird diese Lösung mit wäßriger Salzsäure auf pH 3 eingestellt und 3 mal mit Chloroform extrahiert. Nach der Trocknung über $Na_2SO_4$ erfolgt die Chromatographie an Kieselgel 60 in Chloroform/Methanol (95:5).

Man erhält 477 mg der Verbindung III ($R^1$ = $R^2$ = H, $R^3$ = Dimethyltertiärbutylsilyl, 90 % bezogen auf die eingesetzten Diels-Alder-Addukte).

Beispiel 7
Nachweis der cytotoxischen Wirkung:

Die cytotoxische Wirkung der Verbindungen kann in verschiedenen Testsystemen wie z.B. im Soft-Agar-Kolonietest an Leukämiezellen der Maus in vitro festgestellt werden.

Koloniebildung von L1210-Leukämiezellen in Soft-Agar:

Diese Methode dient zum Nachweis eines Einflusses der Testsubstanzen auf das Wachstumsverhalten der Zellen über mehrere Generationen (bei einer Zellzykluszeit von 10-12 Stunden werden in der Testzeit von 7 Tagen ca. 14 aufeinanderfolgende Generationen beobachtet). Zytostatisch wirksame Substanzen bewirken in diesem Test eine Reduktion der zu beobachtenden Koloniezahl gegenüber einer unbehandelten Kontrolle. Im einzelnen wurde der Test wie folgt durchgeführt:

500 L1210-Leukämiezellen pro Platte werden mit unterschiedlichen Konzentrationen der gemäß Beispiel 3 erhaltenen Verbindung der Formel II als Testsubstanz 1 Stunde bei 37°C inkubiert. Anschließend werden die Zellen zweimal mit McCoy-5A-Medium gewaschen und schließlich in Petrischalen nach Zusatz von 0,3 % Agar ausgegossen. Kontrollen werden lediglich mit frischem Medium inkubiert. Anstelle der einstündigen Inkubation werden außerdem unterschiedliche Konzentrationen der Testsubstanz der oberen Agarschicht zugemischt, um so eine kontinuierliche Exposition der Zellen über die gesamte Inkubationszeit zu erreichen. Nach Erstarren des Agars werden die Platten im Brutschrank 7 Tage bei 37°C inkubiert (5 % $CO_2$, 95 % relative Luftfeuchtig-

0245831

keit). Anschließend wird die Zahl der entstandenen Kolonien mit einem Durchmesser von > 60 µ gezählt. Die Ergebnisse werden ausgedrückt als der Quotient der Koloniezahl in behandelten Agarplatten und der Koloniezahl in der unbehandelten Kontrolle. Aus der so erhaltenen Dosis-Wirkungskurve wurde die $IC_{50}$ als Maß für die Wirksamkeit der Testsubstanz ermittelt. Als Vergleichssubstanz dient bei diesem Test das Zytostatikum Adriamycin. Die Ergebnisse sind in der Tabelle zusammengefaßt.

Tabelle

| Substanz | Stammzellassay cont. $IC_{50}$ (µg/ml) | Stammzellassay 1 h $IC_{50}$ (µg/ml) |
|---|---|---|
| Testsubstanz | 0,04 | 0,26 |
| Streptazolin | 10 | 10 |
| Adriamycin | 0,02 | 0,04 |

Beurteilung:

Wie die Tabelle zeigt ist die gemäß Beispiel 3 erhaltene Verbindung der Formel II hinsichtlich ihrer Zytotoxizität mit Adriamycin vergleichbar, während Streptazolin, die Ausgangsverbindung, keine nennenswerte Zytotoxizität zeigt.

0245831

PATENTANSPRÜCHE: HOE 86/F 109

1. Die Verbindung der allgemeinen Formel I,

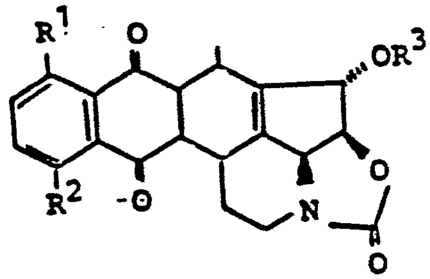

I

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten.

2. Die Verbindung der allgemeinen Formel II,

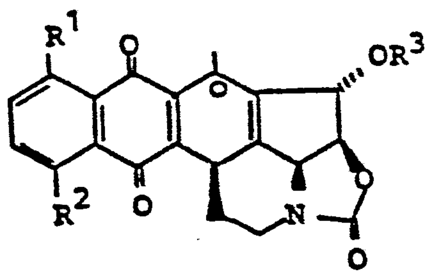

II

bei der an der mit "o" gekennzeichneten Position die beiden möglichen Epimeren auftreten und in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten.

3. Die Verbindung der allgemeinen Formel III,

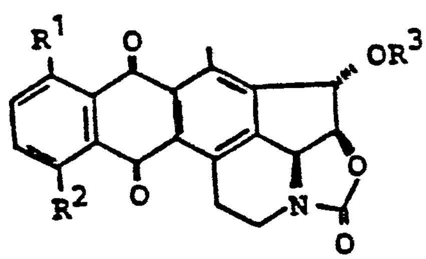

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten.

4. Verfahren zur Herstellung der Verbindung der allgemeinen Formel I,

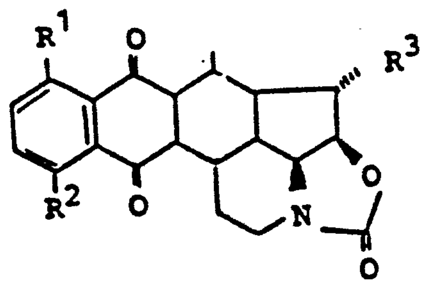

I

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, dadurch gekennzeichnet, daß man die Verbindung der Formel IV

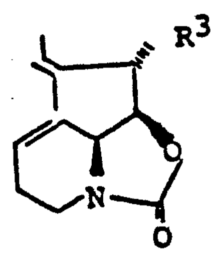

IV

in der $R^3$ die obengenannte Bedeutung hat mit der Verbindung der allgemeinen Formel V,

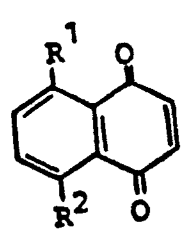

V

in der $R^1$ und $R^2$ die obengenannte Bedeutung haben, miteinander zur Reaktion bringt.

5. Verfahren nach Anspruch 4, dadurch gekennzeichnet, daß man die Reaktion in Toluol bei 100 bis 120°C durchführt.

6. Verfahren nach Anspruch 4, dadurch gekennzeichnet, daß man die Reaktion unter Zusatz von Lewis-säuren bei -20 bis +10°C in Methylenchlorid durchführt.

7. Verfahren zur Herstellung der Verbindungen der allgemeinen Formeln II und III,

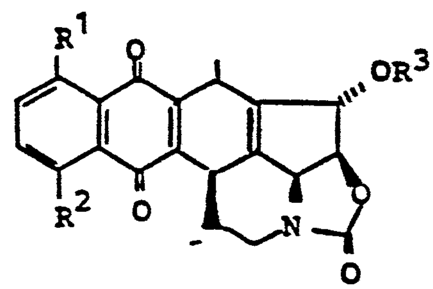

II

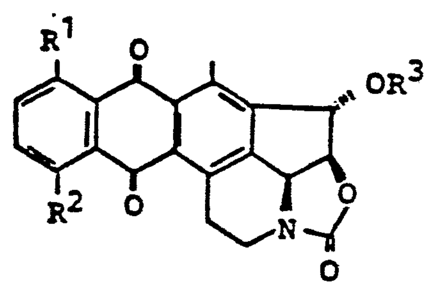

III

in denen $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, dadurch gekennzeichnet, daß die Verbindungen der allgemeinen Formel I,

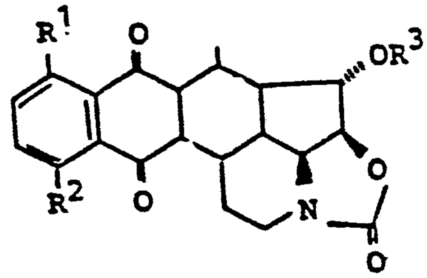

in der $R^1$, $R^2$ und $R^3$ die obengenannte Bedeutung haben, oxidiert wird.

8. Verfahren nach Anspruch 7, dadurch gekennzeichnet, daß die Oxidation mit Luftsauerstoff in einer Mischung aus Wasser und einem $(C_1-C_4)$-Alkohol bei pH-Werten von 9 bis 14 durchgeführt wird.

9. Verfahren nach Anspruch 7, dadurch gekennzeichnet, daß die Oxidation in wasserfreiem Methanol mit Pyridin und reinem Sauerstoff erfolgt.

10. Verwendung der Verbindungen der allgemeinen Formeln I und/oder II und/oder III,

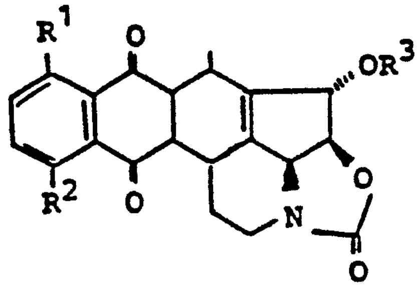

I

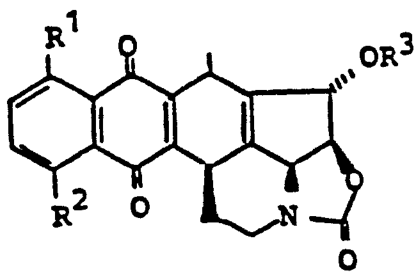

II

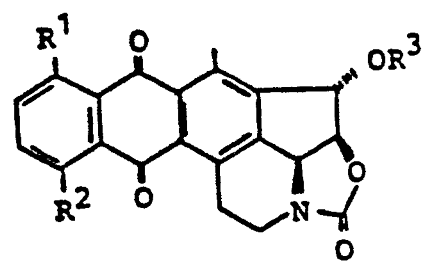

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, als cytotoxisch wirkendes Agens.

11. Verwendung der Verbindungen der allgemeinen Formeln I und/oder II und/oder III,

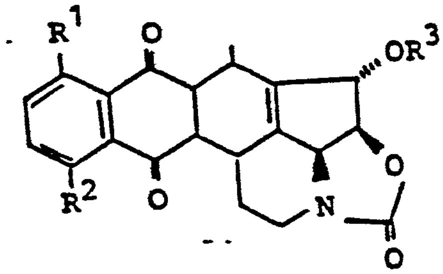

I

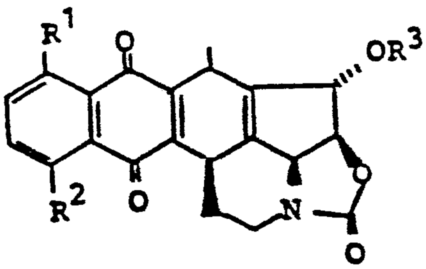

II

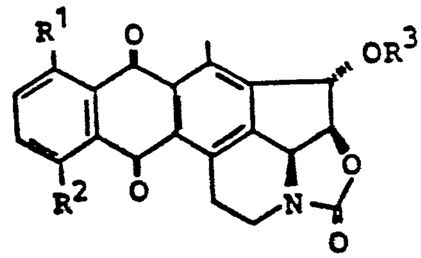

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, als antimikrobiell und gegen Protozoen wirkendes Agens.

12. Verwendung der Verbindung der allgemeinen Formeln I und/oder II und/oder III,

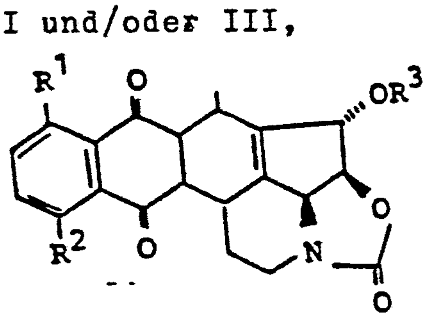

I

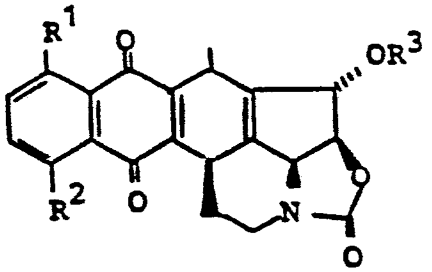

II

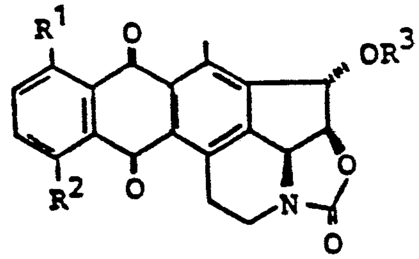

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl, oder Glukosyl bedeuten, zur Herstellung eines Arzneimittels.

13. Verwendung der Verbindung der allgemeinen Formeln I und/oder II und/oder III

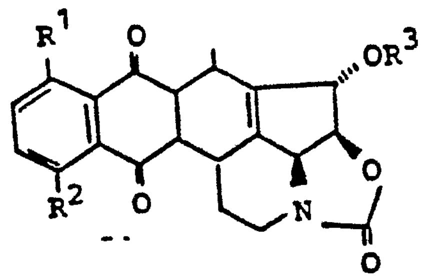

I

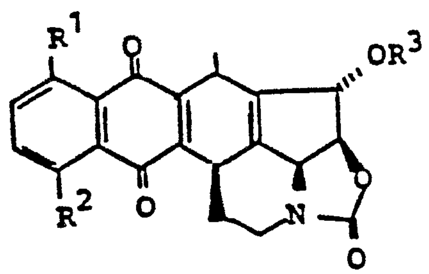

II

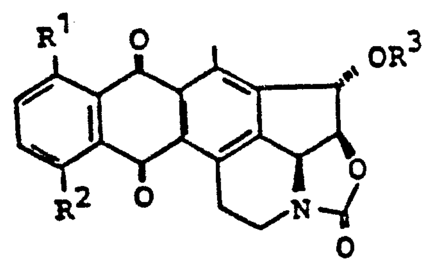

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, zur Herstellung eines Arzneimittels zur Behandlung von infektiösen Krankheiten und Tumoren.

Patentansprüche Österreich und Spanien:

1. Verfahren zur Herstellung der Verbindung der allgemeinen Formel I,

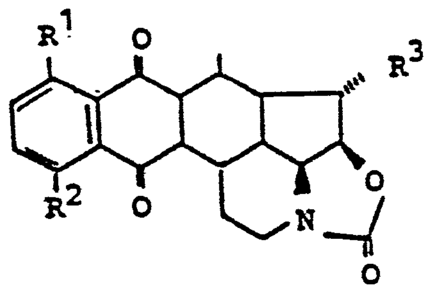

I

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, dadurch gekennzeichnet, daß man die Verbindung der Formel IV

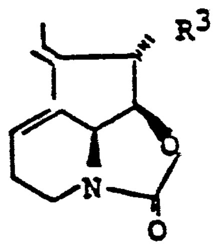

IV

in der $R^3$ die obengenannte Bedeutung hat mit der Verbindung der allgemeinen Formel V,

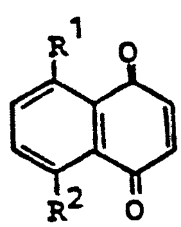

V

in der $R^1$ und $R^2$ die obengenannte Bedeutung haben, miteinander zur Reaktion bringt.

2. Verfahren nach Anspruch 1, dadurch gekennzeichnet, daß man die Reaktion in Toluolbei 100 bis 120°C durchführt.

3. Verfahren nach Anspruch 1, dadurch gekennzeichnet, daß man die Reaktion unter Zusatz von Lewis-säuren bei -20 bis +10°C in Methylenchlorid durchführt.

4. Verfahren zur Herstellung der Verbindungen der allgemeinen Formeln II und III,

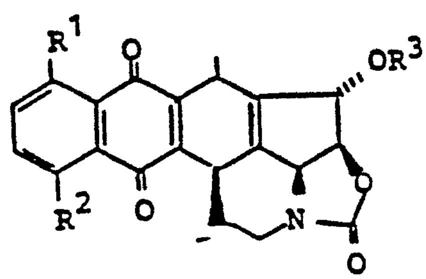

II

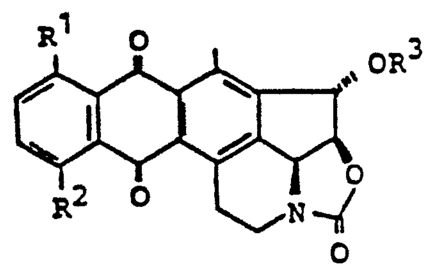

III

in denen $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, dadurch gekennzeichnet, daß die Verbindungen der allgemeinen Formel I,

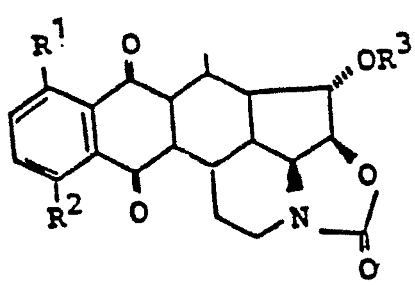

in der $R^1$, $R^2$ und $R^3$ die obengenannte Bedeutung haben, oxidiert wird.

5. Verfahren nach Anspruch 4, dadurch gekennzeichnet, daß die Oxidation mit Luftsauerstoff in einer Mischung aus Wasser und einem $(C_1-C_4)$-Alkohol bei pH-Werten von 9 bis 14 durchgeführt wird.

6. Verfahren nach Anspruch 4, dadurch gekennzeichnet, daß die Oxidation in wasserfreiem Methanol mit Pyridin und reinem Sauerstoff erfolgt.

7. Verwendung der Verbindungen der allgemeinen Formeln I, II und III,

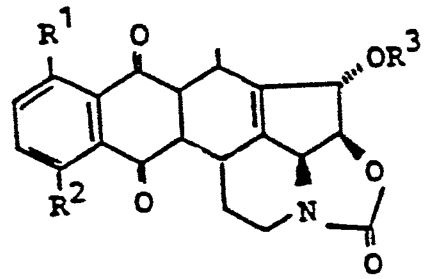

I

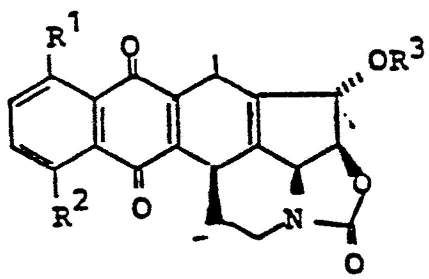

II

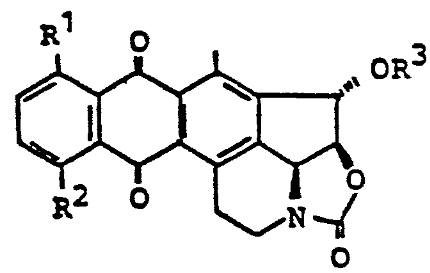

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, als cytotoxisch wirkendes Agens.

8. Verwendung der Verbindungen der allgemeinen Formeln I, II und III,

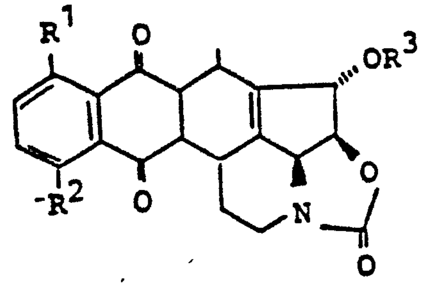

I

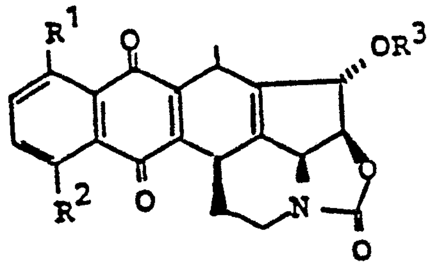

II

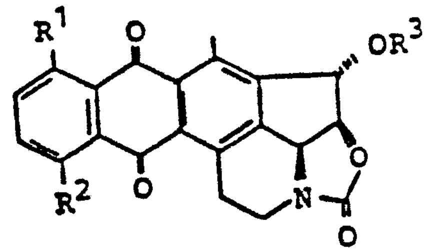

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, als antimikrobiell und gegen Protozoen wirkendes Agens.

0245831

Patentansprüche Griechenland:

HOE 86/F 109

1. Die Verbindung der allgemeinen Formel I,

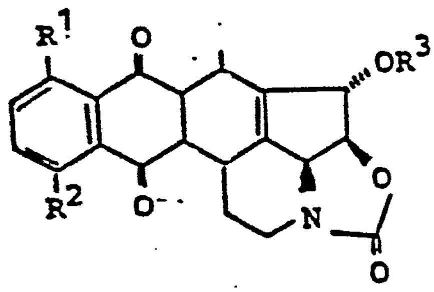

I

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten.

2. Die Verbindung der allgemeinen Formel II,

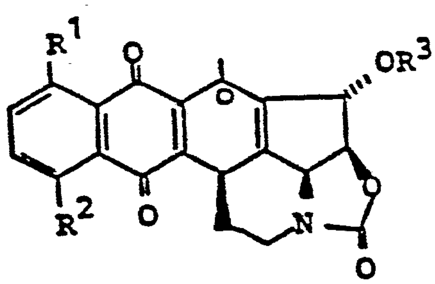

II

bei der an der mit "o" gekennzeichneten Position die beiden möglichen Epimeren auftreten und in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten.

3. Die Verbindung der allgemeinen Formel III,

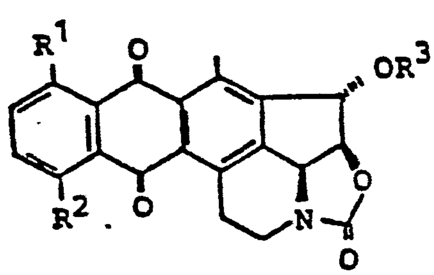

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten.

4. Verfahren zur Herstellung der Verbindung der allgemeinen Formel I,

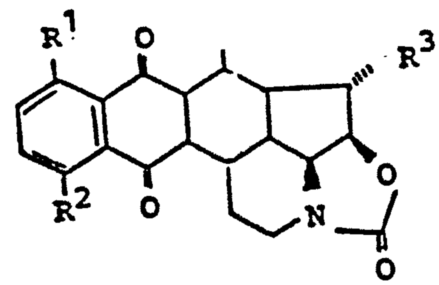

I

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, dadurch gekennzeichnet, daß man die Verbindung der Formel IV

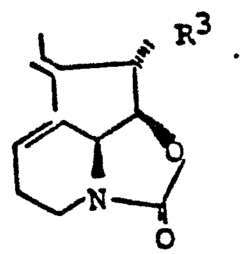

IV

in der $R^3$ die obengenannte Bedeutung hat mit der Verbindung der allgemeinen Formel V,

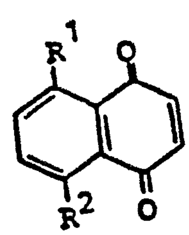

V

in der $R^1$ und $R^2$ die obengenannte Bedeutung haben, miteinander zur Reaktion bringt.

5. Verfahren nach Anspruch 4, dadurch gekennzeichnet, daß man die Reaktion in Toluol bei 100 bis 120°C durchführt.

6. Verfahren nach Anspruch 4, dadurch gekennzeichnet, daß man die Reaktion unter Zusatz von Lewis-säuren bei -20 bis +10°C in Methylenchlorid durchführt.

7. Verfahren zur Herstellung der Verbindungen der allgemeinen Formeln II und III,

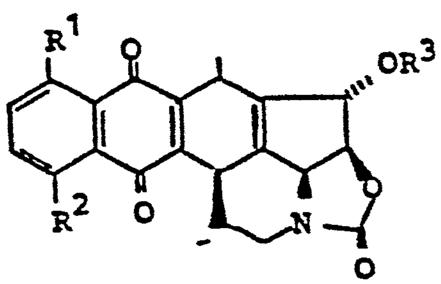

II

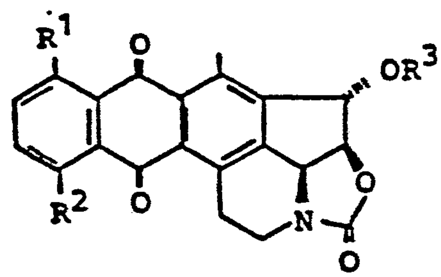

III

in denen $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, dadurch gekennzeichnet, daß die Verbindungen der allgemeinen Formel I,

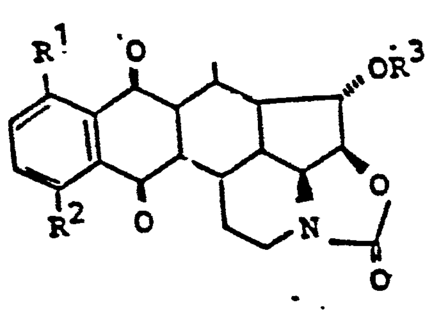

in der $R^1$, $R^2$ und $R^3$ die obengenannte Bedeutung haben, oxidiert wird.

8. Verfahren nach Anspruch 7, dadurch gekennzeichnet, daß die Oxidation mit Luftsauerstoff in einer Mischung aus Wasser und einem $(C_1-C_4)$-Alkohol bei pH-Werten von 9 bis 14 durchgeführt wird.

9. Verfahren nach Anspruch 7, dadurch gekennzeichnet, daß die Oxidation in wasserfreiem Methanol mit Pyridin und reinem Sauerstoff erfolgt.

10. Verwendung der Verbindungen der allgemeinen Formeln I und/oder II und/oder III,

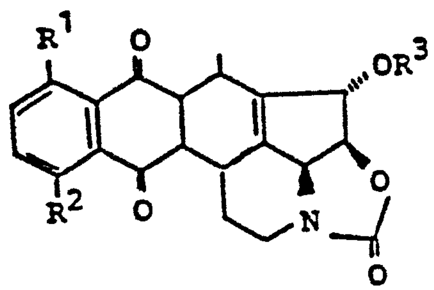

I

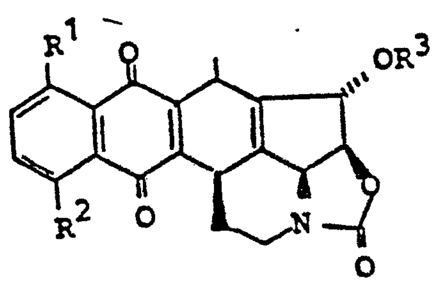

II

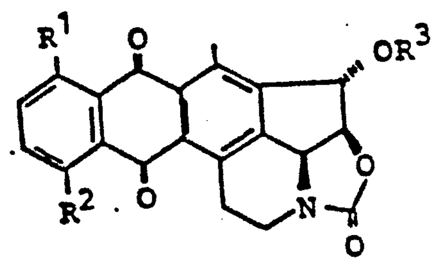

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, als cytotoxisch wirkendes Agens.

11. Verwendung der Verbindungen der allgemeinen Formeln I und/oder II und/oder III,

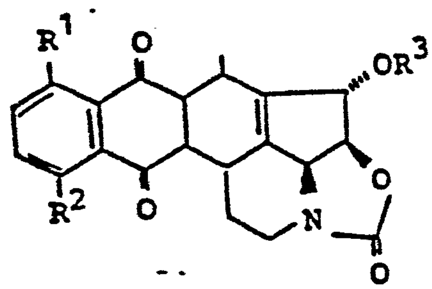

I

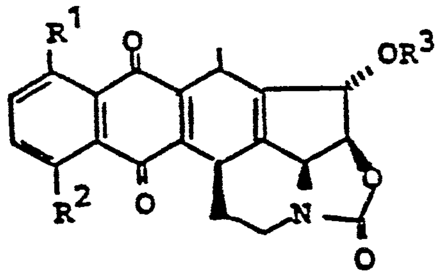

II

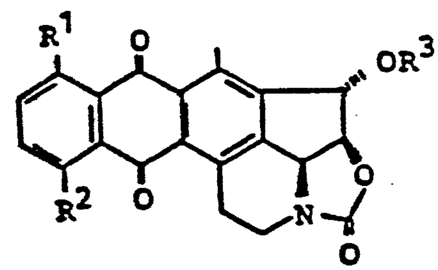

III

in der $R^1$ und $R^2$ unabhängig voneinander Wasserstoff, Hydroxyl, Methoxy, O-Acetyl oder O-Benzyl und $R^3$ Wasserstoff, Acetyl, Trimethylsilyl, Dimethyltertiärbutylsilyl oder Glukosyl bedeuten, als antimikrobiell und gegen Protozoen wirkendes Agens.

FIG. 1

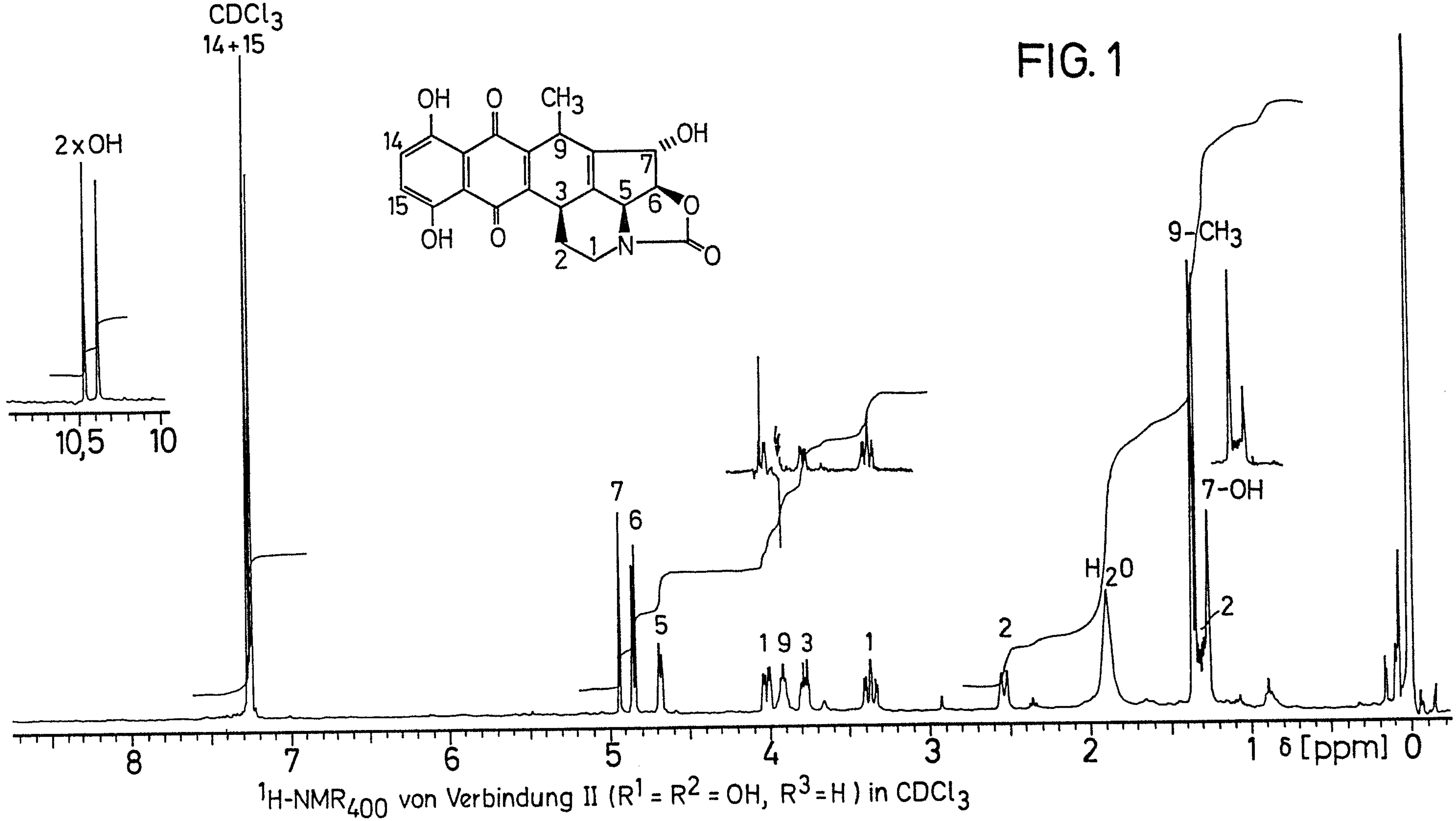


$^1$H-NMR$_{400}$ von Verbindung II ($R^1 = R^2 = OH$, $R^3 = H$) in $CDCl_3$

FIG. 2

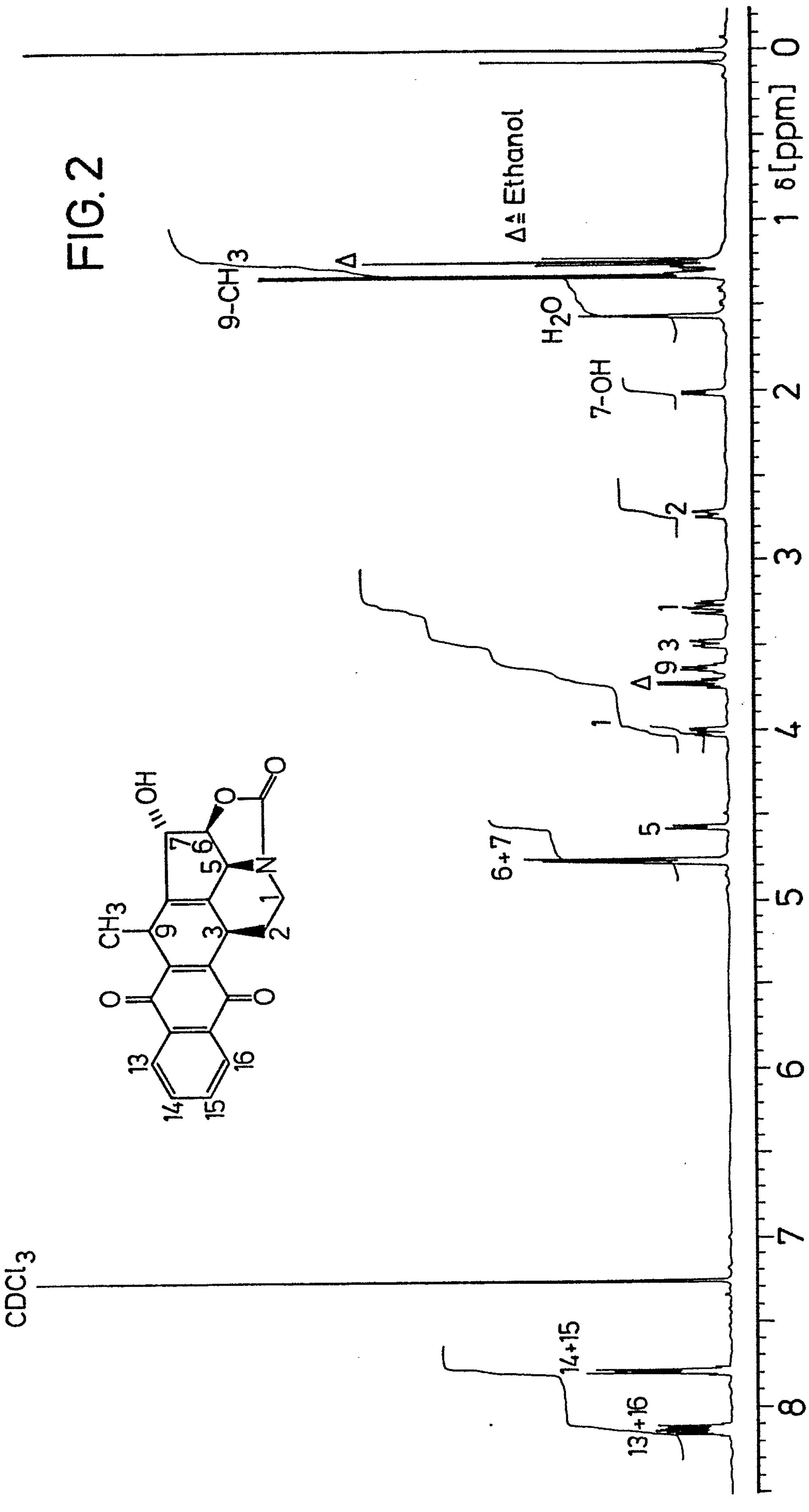


$^1$H-NMR$_{400}$ von Verbindung II ($R^1 = R^2 = R^3 = H$) in $CDCl_3$

FIG. 3

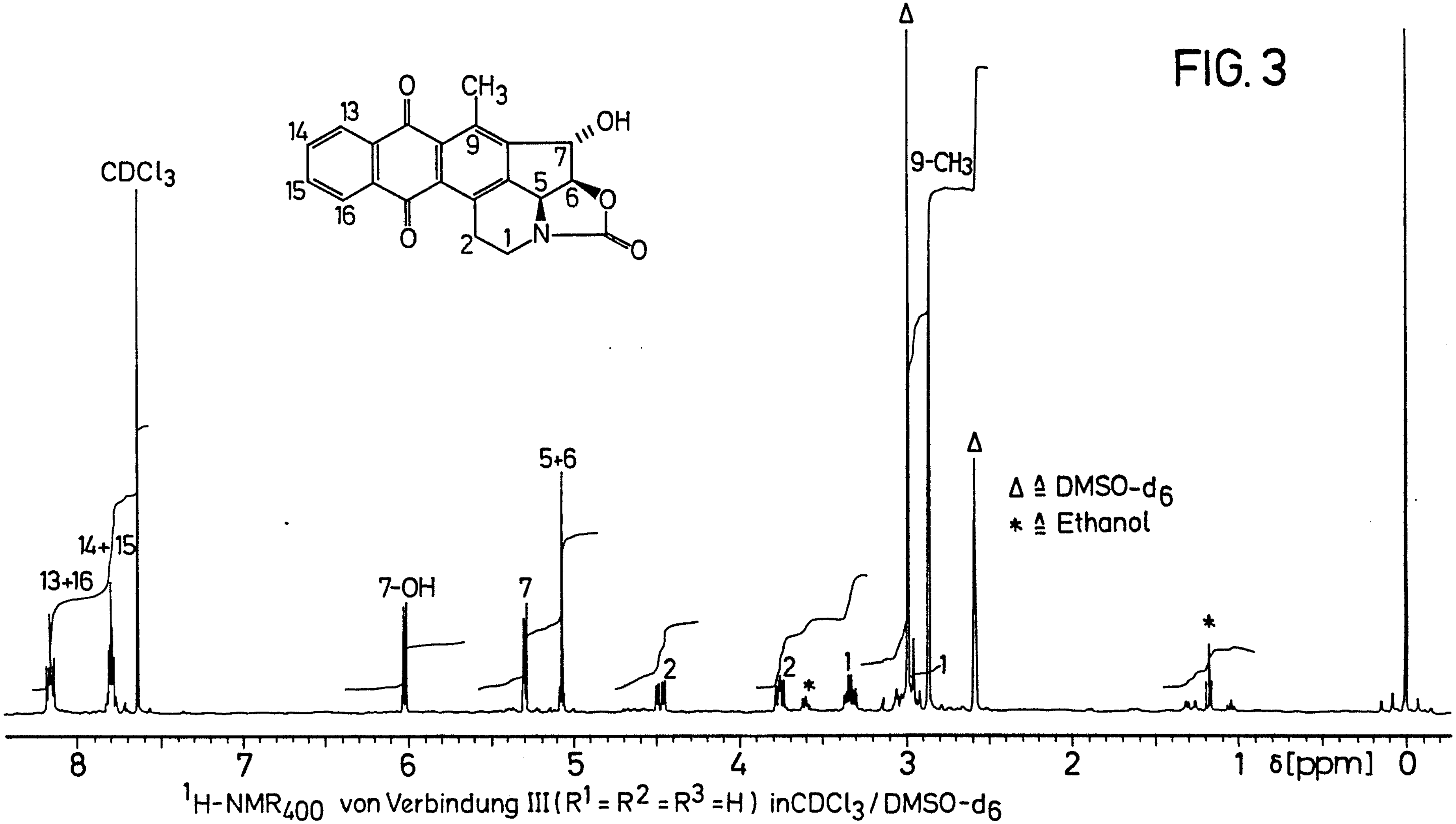


$^1$H-NMR$_{400}$ von Verbindung III ($R^1 = R^2 = R^3 = H$) in $CDCl_3$ / DMSO-$d_6$